# EUROPÄISCHE PATENTSCHRIFT

(11) **EP 0 521 965 B1**
(45) Veröffentlichungstag und Bekanntmachung des Hinweises auf die Patenterteilung: **28.12.1994**
(21) Anmeldenummer: 91906660.5
(22) Anmeldetag: 18.03.1991
(51) Int. Cl.: C11D 1/83, C11D 1/94, A61K 7/50, A61K 7/08

(54) **WÄSSRIGE DETERGENSZUBEREITUNG**
AQUEOUS DETERGENT COMPOSITION
COMPOSITION DETERGENTE AQUEUSE

(30) Priorität: 26.03.1990 DE 4009616
(43) Veröffentlichungstag der Anmeldung: 13.01.1993
(73) Patentinhaber: Henkel Kommanditgesellschaft auf Aktien, 40191 Düsseldorf (DE)
(72) Erfinder: SCHOLZ, Wolfhard, D-4150 Krefeld 11 (DE); SCHOSSER, Gryta, D-4150 Krefeld (DE); SCHNEIDER, Werner, Dr., D-4150 Krefeld (DE); SCHELGES, Heike, D-4150 Krefeld (DE); WALDMANN-LAUE, Marianne, Dr., D-4018 Langenfeld (DE)
(86) Internationale Anmeldenummer: EP9100516
(87) Internationale Veröffentlichungsnummer: WO9114761

(56) Entgegenhaltungen:
- EP-A- 0 133 345
- EP-A- 0 203 750
- EP-A- 0 339 498
- EP-A- 0 356 784
- DE-A- 2 252 281
- US-A- 4 668 422

## Beschreibung

Die Erfindung betrifft wäßrige Detergenszubereitungen, die besonders geeignet sind als flüssige oder pastöse Körperreinigungsmittel und die eine erhöhte Viskosität, eine gute Verdickbarkeit durch Zusatz wasserlöslicher Elektrolytsalze und ein verbessertes Schäumvermögen aufweisen, und die eine Kombination Von Eiweißhydrolysat-Fettsäure-Kondensationsprodukten (EFK) und Alkylglykosiden enthalten.

Die Eiweiß-Fettsäure-Kondensationsprodukte sind eine seit langem bekannte und wegen ihrer bekannten Hautverträglichkeit für kosmetische Reinigungsmittel empfohlene Tensidklasse. Sie weisen jedoch nur sehr geringe Schaumkraft auf und ihre wäßrigen Lösungen lassen sich durch Elektrolytzusatz praktisch nicht verdicken. Sie müssen daher mit schaumstarken und gut verdickbaren Aniontensiden kombiniert werden, wodurch die gute Hautverträglichkeit wieder gemindert wird. Es bestand daher die Aufgabe, für Tenside vom Typ der Eiweiß-Fettsäure-Kondensationsprodukte einen geeigneten Tensidpartner zu finden, der ohne Verringerung der Verträglichkeit eine Verbesserung der Schäumeigenschaften und eine leichte Viskositätsanhebung ermöglicht. Diese Eigenschaften sind für kosmetische Körperreinigungsmittel wie z. B. flüssige Seifen, Duschbäder, Badepräparate, kosmetische Waschpasten, Haarspülungen und Haarshampoos besonders wichtig, da diese Produkte von Hand auf dem Kopf oder Körper verteilt werden und daher eine gewisse Viskosität benötigen, um nicht wasserdünn zwischen den Fingern hindurchzulaufen. Aber auch bei flüssigen Feinwasch- und Geschirrspülmitteln sind solche Eigenschaften durchaus erwünscht.

Aus US-A-4 668 422 waren flüssige Zubereitungen mit einem Alkylglycosid, einem Amphotensid und einem nichtionischen Schaumbooster bekannt. EP-A-356 784 beschreibt flüssige Reinigungsmittel, die ein Fettsäure-Asparaginsäure-Kondensat und ein Imidazoliniumtensid enthalten. EP-A-339 498 betrifft eine Tensidkombination aus einem Fettsäurekondensat einer sauren Aminosäure und einem Olefinsulfonat für flüssige Reinigungsmittel. Aus EP-A-133 345 war eine flüssige Seife bekannt, die neben anionischen und nichtionischen Tensiden ein wasserlösliches verdickendes Polymer enthält. EP-A-203 750 beschreibt hautmilde Körperreinigungsmittel, die milde synthetische Tenside, Feuchthaltemittel, Polymere und Seife enthalten können.

Es wurde nunmehr gefunden, daß durch Kombination von Eiweiß-Fettsäure-Kondensationsprodukten mit Alkylglycosiden sich besonders interessante wäßrige Detergenszubereitungen herstellen lassen, die nicht nur die Vorteile beider Tensidklassen vereinen, sondern die darüberhinaus neue, unerwartete Eigenschaften aufweisen.

Gegenstand der Erfindung sind daher wäßrige Detergenszubereitungen, die
(A) 1 - 20 Gew.-% eines Kondensationsproduktes von Fettsäuren mit 12 - 22 C-Atomen und Aminosäuren, wasserlöslichen Oligo- und/oder Polypeptiden oder dessen wasserlösliches Salz und
(B) 1 - 20 Gew.-% eines Alkylglycosids der Formel R¹-(G)ₓ, in der R¹ eine Alkylgruppe mit 8 - 22 C-Atomen und (G)ₓ ein Glucosid- oder Oligoglycosidrest mit einem Oligomerisationsgrad x = 1 - 10 ist,
enthalten. Die erfindungsgemäßen Detergenszubereitungen weisen eine höhere Viskosität - oder bei niedrigeren Tensidkonzentrationen eine bessere Verdickbarkeit durch Zusatz von Elektrolyten - auf als Zubereitungen, die nur eine der beiden Komponenten enthalten. Darüber hinaus ist das Schäumvermögen der erfindungsgemäßen Zubereitungen synergistisch gesteigert.

Kondensationsprodukte von Fettsäuren und Aminosäuren sind z. B. die literaturbekannten N - C₁₂ - C₁₈ - Acylsarkosine, das N - Lauroylglycin, das N - Lauroylalanin, die N - C₁₂ - C₁₈ -Acylasparaginsäure und die N - C₁₂ - C₈ - Acyl-glutaminsäure. Kondensationsprodukte von Fettsäuren und Oligo- oder Polypeptiden sind vor allem die bekannten Kondensationsprodukte von Fettsäuren und Proteinhydrolysaten. Zu diesem Zweck werden Proteine, z. B. Sojaprotein, Collagen oder Keratin zunächst einer Hydrolyse unterworfen, die zu wasserlöslichen Oligopeptiden und/oder Polypeptiden führt. Diese werden dann mit Fettsäurechloriden acyliert. Man kann aber auch, z. B. gemäß FR-PS-936632, das Alkalisalz des Peptids mit der Fettsäure-Seife auf 130 - 220°C erhitzen. Die auf diese Weise erhältlichen Salze der Proteinhydrolysat-Fettsäure-Kondensationsprodukte sind im Handel unter der Bezeichnung Lamepon ^{R} oder Maypon ^{R} in Form von wäßrigen Lösungen erhältlich.

Alkylglycoside der Formel R¹-(G)ₓ sind seit langem bekannte oberflächenaktive Stoffe, die aus Zuckern und aliphatischen, primären Alkoholen mit 8 - 22 C-Atomen unter Acetalisierung herstellbar sind. Als Zuckerkomponenten (Glycosen) kommen bevorzugt Glucose, daneben aber auch Fructose, Mannose, Galactose, Talose, Gulose, Allose, Altrose, Idose, Arabinose, Xylose, Lyxose, Ribose und Gemische davon infrage. Bevorzugt wegen der guten Verfügbarkeit und der guten Anwendungseigenschaften sind die Acetalisierungsprodukte der Glucose mit Fettalkoholen (R¹-OH), die z. B. aus natürlichen Ölen und Fetten nach bekannten Verfahren erhältlich sind, insbesondere mit linearen, primären, gesättigten und ungesättigten Fettalkoholen mit 8 - 22 C-Atomen.

Alkylglycoside der Formel R¹-(G)ₓ, ihre Herstellung und Verwendung als oberflächenaktive Stoffe sind beispielsweise aus US-A-3,839,318, US-A-3,707,535, US-A-3,547,828, DE-A-19 43 689, DE-A-20 364 72, DE-A-30 01 064 und EP-A-77 167 bekannt.

Bezüglich des Glycosidrestes -(G)ₓ gilt, daß sowohl Monoglycoside (x = 1), bei denen ein Zuckerrest glycosidisch mit dem Fettalkohol verbunden ist, als auch oligomere Glycoside mit einem Oligomerisationsgrad x = 2 - 10 geeignet sind. Gewöhnlich liegen Gemische von Mono- und Oligoglycosiden vor. Bevorzugt geeignet sind Alkylglycoside (B) der Formel R¹-(G)ₓ, in welchen R¹ eine Alkylgruppe mit 8 - 18 C-Atomen und (G)ₓ ein Glucosid- oder Oligoglucosid mit einem Oligomerisationsgrad x = 1 - 4 ist. Ganz besonders bevorzugt ist R¹, eine Alkylgruppe mit 12 - 16 C-Atomen, und -(G)ₓ, der Rest eines Gemisches von Gucosiden und Oligoglucosiden mit einem mittleren Oligomerisationsgrad x = 1 - 2.

Die erfindungsgemäßen wäßrigen Detergenszubereitungen können neben den obligatorischen Komponenten (A) und (B) auch noch weitere Tenside und Zusätze enthalten, es sollten allerdings nicht mehr als 20 Gew.-% weiterer oberflächenaktiver Stoffe enthalten sein.

Wenn der Gehalt der Komponenten (A) und (B) insgesamt unter 20 Gew.-% und der Gehalt an Komponente (B) unter 8 Gew.-% ist, kann die Viskosität erfindungsgemäßer Detergenszubereitungen noch unbefriedigend sein. Es ist aber ein besonderer Vorteil der erfindungsgemäßen Zubereitungen, daß sich in solchen Fällen die Viskosität leicht durch Zusatz von wasserlöslichen anorganischen Elektrolytsalzen oder auch durch Zusatz eines amphoteren oder zwitterionischen Tensids oder durch Zusatz beider Produkte anheben läßt.

In einer bevorzugten Ausführung enthalten die erfindungsgemäßen Detergenszubereitungen daher zusätzlich
(C) 1 - 8 Gew.-% eines amphoteren oder zwitterionischen Tensids und/oder
(D) 0,1 - 5 Gew.-% eines wasserlöslichen, anorganischen Elektrolytsalzes
Amphotere Tenside (C) sind gekennzeichnet durch eine lipophile Alkyl- oder Acylgruppe und eine Carboxylgruppe. Beispiele für geeignete amphotere Tenside sind z. B. N - (C₁₂-C₁₈) - alkylaminoessigsäure, N - (C₁₂-C₁₈) - acylaminopropyl - aminopropionsäure. Zwitterionische Tenside zeichnen sich durch eine lipophile Alkyl- oder Acylgruppe mit 8 - 18 C-Atomen, eine quartäre Ammoniumgruppe und eine Carboxylgruppe aus. Beispiele für geeignete zwitterionische Tenside (C) sind z. B. N - (C₁₂-C₁₈) - alkyl - N, N - dimethyl - glycinat, N - (C₁₂-C₁₈) - acylaminopropyl - N, N - dimethyl - glycinat, N - (C₁₂-C₁₈) - acyl - aminoethyl - N, N - dimethyl - glycinat, N - (C₁₂-C₁₈) - acylaminopropyl - N - methyl - N - hydroxyethyl - glycinat, 2 - (C₁₂-C₁₈) - alky - 1 -carboxymethyl - 3 - hydroxylethyl - imidazolin.

Als anorganische Elektrolytsalze (D) eignen sich alle wasserlöslichen Alkali-, Ammonium- und Erdalkalisalze, z. B. die Fluoride, Chloride, Bromide, Sulfate, Phosphate, Nitrate und Hydrogencarbonate, soweit sie in einer Menge von wenigstens 1 Gew.-% bei 20°C in Wasser löslich sind. Bevorzugt werden die Chloride oder Sulfate eines Alkalimetalls oder des Magnesiums verwendet; besonders wirksam sind Natriumchlorid und Magnesiumchlorid.

Die rheologischen Eigenschaften der erfindungegemäßen Detergenszusammensetzungen sind vor allem dann besonders gut, wenn die Summe der Komponenten (A) + (B) + (C) + (D) wenigstens 8 Gew.-% der gesamten Zusammensetzung beträgt.

Die erfindungsgemäßen Zubereitungen können darüberhinaus weitere Komponenten enthalten, die sie für den jeweiligen Anwendungszweck besonders geeignet machen. So können z. B. weitere nichtionische oder anionische Tenside enthalten sein; deren Menge ist bevorzugt so bemessen, daß neben (A) und (B) nicht mehr als 20 Gew.-% weiterer oberflächenaktiver Stoffe zugegen sind.

In untergeordneten Mengen können schließlich Duftstoffe, Farbstoffe, Trübungs- und Perlglanzmittel, antimikrobielle Stoffe, Konservierungsmittel, Antioxidantien, hautkosmetische Wirkstoffe, Pflanzenextrakte, Proteinhydrolysate, Puffersubstanzen, Komplexbildner, wasserlösliche Polymere, Schichtsilikate und andere bekannte Hilfs- und Zusatzstoffe enthalten sein, wie sie in Shampoos, Badezusätzen, Duschbadpräparaten, flüssigen Seifen, flüssigen oder pastösen Hautreinigungsmitteln, flüssigen Haarspülmitteln sowie in flüssigen Feinwasch- und Geschirrspülmitteln und flüssigen Haushaltsreinigern üblich sind.

Die folgenden Beispiele sollen den Gegenstand der Erfindung näher erläutern:

### Beispiele

Es wurden folgende Prüfsubstanzen verwendet:
- EFK =: Kollagenhydrolysat - Kokosfettsäure - (C₁₂-C₁₈) - kondensationsprodukt, Na-Salz (es wurde das Handelsprodukt Lamepon ^{R} S als 30%ige wäßrige Lösung verwendet).
- APG =: Alkyl - (C₁₂₋₁₄) - glucosid (Die Alkylgruppe entspricht der C-Kettenverteilung einer C₁₂-C₁₄ - Kokosfettalkohol - Fraktion; der mittlere Oligomerisationsgrad x = 1,4), als 50%ige wäßrige Lösung.

Die Prüfrezepturen 1 - 10 der Tabellen I und II wurden durch Vermischen der Komponenten bei 20°C hergestellt.

### Ergebnisse:

Die Viskosität der Rezepturen wurde bei 20°C mit Hilfe eines Haake Rotationsviskosimeters (Type RV3) gemessen. Die Ergebnisse sind aus Tabelle I zu entnehmen.

Das Schäumvermögen der Rezepturen wurde mit einem sog. standardisierten Wannentest ermittelt. Hierzu wurde eine 0,01%ige Lösung der Prüfrezeptur in einer Wanne hergestellt (15 g auf 150 l Wasser von 20°C) und durch einlaufendes Wasser aufgeschäumt. Der unter definierten Bedingungen (Zeit, Wasserdruck) gebildete Schaum wurde volumetrisch gemessen. Das Ergebnis ist der Tabelle II zu entnehmen.

**Tabelle 1**

| Zusammensetzung* | 1 | 2 | 3 | 4 | 5 | 6 | 7 | 8 |
|---|---|---|---|---|---|---|---|---|
| EFK | 15 | 15 | 7,5 | 9 | 10 | 10 | 12 | 12 |
| APG | - | - | 12,5 | 10 | 8,5 | 8,5 | 5 | 5 |
| NaCl | - | 1-3 | - | - | - | 0,5 | - | 2 |
| H₂O | 85 | 82-84 | 80 | 81 | 81 | 81 | 83 | 81 |
| Viskosität (20 °C, Pa·s) | unter 0,1 | unter 0,1 | 4,95 | 14,3 | 4,35 | 13,35 | unter 0,1 | 2,55 |
| | | | | | | | | |

| Zusammensetzung* | 9 | 10 | 11 | 12 | 13 | 14 | 15 | 16 |
|---|---|---|---|---|---|---|---|---|
| EFK | 8 | 8 | 4 | 4 | 5 | 5 | 3 | 3 |
| APG | - | - | 4 | 4 | 3 | 3 | 5 | 5 |
| NaCl | - | 1-5 | - | 1,5 | - | 2 | - | 1,2 |
| H₂O | 92 | 87-91 | 92 | 90,5 | 92 | 90 | 92 | 90,8 |
| Viskosität (20 °C, Pa·s) | unter 0,1 | unter 0,1 | unter 0,1 | 1,4 | unter 0,1 | 2,7 | unter 0,1 | 8,2 |

| | | | | | | | | |
|---|---|---|---|---|---|---|---|---|
| * in Gew.-% Aktivsubstanz | | | | | | | | |

**Tabelle 2**

| Zusammensetzung* | 17 | 18 | 19 | 3 | 4 |
|---|---|---|---|---|---|
| EFK | 20 | - | 6 | 7,5 | 9 |
| APG | - | 25 | 15 | 12,5 | 10 |
| H₂O | 80 | 75 | 79 | 80 | 81 |
| Schaummenge [l] bei 20 °C | 2 | 13 | 18 | 21 | 23 |
| | | | | | |

| Zusammensetzung* | 20 | 9 | 11 | 13 | 15 |
|---|---|---|---|---|---|
| EFK | - | 8 | 4 | 5 | 3 |
| APG | 8 | - | 4 | 3 | 5 |
| H₂O | 92 | 92 | 92 | 92 | 92 |
| Schaummenge [l] bei 20 °C | 10 | 1 | 19 | 16 | 20 |

| | | | | | |
|---|---|---|---|---|---|
| * in Gew.-% Aktivsubstanz | | | | | |

## Patentansprüche

1. Wäßrige Detergenszubereitung, gekennzeichnet durch einen Gehalt von
(A) 1 - 20 Gew.-% eines Kondensationsproduktes von Fettsäuren mit 12 - 22 C-Atomen und Aminosäuren, wasserlöslichen Oligo- und/oder Polypeptiden oder dessen wasserlösliches Salz und
(B) 1 - 20 Gew.-% eines Alkylglycosids der Formel R¹ - (G)ₓ, in der R¹ eine Alkylgruppe mit 8 - 22 C-Atomen und (G)ₓ eine Glycosid- oder Oligoglycosidrest mit einem Oligomerisationsgrad x = 1 - 10 ist.

2. Wäßrige Detergenszubereitung nach Anspruch 1, dadurch gekennzeichnet, daß die Summe aus dem Kondensationsprodukt (A) und dem Alkylglycosid (B) wenigstens 5 Gew.-% beträgt und daneben nicht mehr als 20 Gew.-% weiterer oberflächenaktiver Stoffe enthalten sind.

3. Wäßrige Detergenszubereitung nach Anspruch 1 oder 2, dadurch gekennzeichnet, daß zusätzlich
(C) 1 - 8 Gew.-% eines amphoteren oder zwitterionischen Tensids und/oder
(D) 0,1 - 5 Gew.-% eines wasserlöslichen anorganischen Elektrolytsalzes enthalten sind.

4. Wäßrige Detergenszusammensetzung nach Anspruch 3, dadurch gekennzeichnet, daß die Summe der Komponenten (A) + (B) + (C) + (D) wenigstens 8 Gew.-% beträgt.

5. Wäßrige Detergenszusammensetzung nach einem der Ansprüche 1 - 4, dadurch gekennzeichnet, daß (A) das Kondensationsprodukt von C₁₂ - C₁₈ - Fettsäuren und einem wasserlöslichen Proteinhydrolysat ist.

6. Wäßrige Detergenszubereitung nach einem der Ansprüche 1 - 5, dadurch gekennzeichent, daß (B) ein Alkylglycosid der Formel R¹-(G)ₓ ist, in der R¹ eine Alkylgruppe mit 12 - 16 C-Atomen und (G)ₓ der Rest eines Gemisches von Glucosiden und Oligoglucosiden mit einem mittleren Oligomerisationsgrad von 1 - 2 ist.

## Claims

1. An aqueous detergent preparation, characterized by a content of
(A) 1 to 20% by weight of a condensation product of fatty acids containing 12 to 22 carbon atoms and amino acids, water-soluble oligo- and/or polypeptides or a water-soluble salt thereof and
(B) 1 to 20% by weight of an alkyl glycoside corresponding the formula R¹-(G)ₓ, in which R¹ is an alkyl group containing 8 to 22 carbon atoms and (G)ₓ is a glycoside or oligoglycoside unit having a degree of oligomerization x of 1 to 10.

2. A water-based detergent preparation as claimed in claim 1, characterized in that the sum of the condensation product (A) and the alkyl glycoside (B) is at least 5% by weight and, in addition, no more than 20% by weight other surface-active agents are present.

3. A water-based detergent preparation as claimed in claim 1 or 2, characterized in that
(C) 1 to 8% by weight of an amphoteric or zwitterionic surfactant and/or
(D) 0.1 to 5% by weight of a water-soluble inorganic electrolyte salt
are additionally present.

4. A water-based detergent preparation as claimed in claim 3, characterized in that the sum of components (A) + (B) + (C) + (D) is at least 8% by weight.

5. A water-based detergent preparation as claimed in any of claims 1 to 4, characterized in that (A) is the condensation product of C₁₂₋₁₈ fatty acids and a water-soluble protein hydrolyzate.

6. A water-based detergent preparation as claimed in any of claims 1 to 5, characterized in that (B) is an alkyl glycoside corresponding to the formula R¹-(G)ₓ, in which R¹ is a C₁₂₋₁₆ alkyl group and (G)ₓ is the residue of a mixture of glucosides and oligoglucosides having an average degree of oligomerization of 1 to 2.

## Revendications

1. Préparation aqueuse de détergent, caractérisée par une teneur en
(A) 1 - 20% en poids d'un produit de condensation d'acides gras contenant de 12 à 22 atomes de carbone et d'aminoacides, d'oligo- et/ou de polypeptides hydrosolubles, ou de son sel hydrosoluble, et
(B) 1 - 20% en poids d'un alkylglycoside de formule R¹⁻(G)ₓ, dans laquelle R¹ représente un groupe alkyle contenant de 8 à 22 atomes de carbone et (G)ₓ représente un résidu glycoside ou oligoglycoside ayant un degré d'oligomérisation x = 1 - 10.

2. Préparation aqueuse de détergent selon la revendication 1, caractérisée en ce que la somme du produit de condensation (A) et de l'alkylglycoside (B) s'élève au moins à 5% en poids, la préparation ne contenant en outre pas plus de 20% en poids d'autres substances tensioactives.

3. Préparation aqueuse de détergent selon la revendication 1 ou 2, caractérisée en ce qu'elle contient en outre
(C) de 1 à 8% en poids d'un agent tensioactif amphotère ou zwittérionique et/ou
(D) de 0,1 à 5% en poids d'un sel électrolytique inorganique hydrosoluble.

4. Composition aqueuse de détergent selon la revendication 3, caractérisée en ce que la somme des composants (A) + (B) + (C) + (D) s'élève à au moins 8% en poids.

5. Composition aqueuse de détergent selon l'une des revendications 1 à 4, caractérisée en ce que (A) est le produit de condensation d'acides gras en C₁₂-C₁₈ et d'un hydrolysat protéinique hydrosoluble.

6. Préparation aqueuse de détergent selon l'une des revendications 1 à 5, caractérisée en ce que (B) est un alkylglycoside de formule R¹-(G)ₓ, dans laquelle R¹ représente un groupe alkyle contenant de 12 à 16 atomes de carbone et (G)ₓ représente le résidu d'un mélange de glycosides et d'oligoglycosides, ayant un degré d'oligomérisation moyen de 1 - 2.
